# EUROPÄISCHE PATENTSCHRIFT

(11) **EP 0 957 958 B1**
(45) Veröffentlichungstag und Bekanntmachung des Hinweises auf die Patenterteilung: **01.10.2003**
(21) Anmeldenummer: 96918574.3
(22) Anmeldetag: 02.07.1996
(51) Int. Cl.: A61M 5/32, A61M 5/50

(54) **AUTOMATISCHE KANÜLENRÜCKZUGSVORRICHTUNG FÜR INJEKTIONSSPRITZEN**
AUTOMATIC CANNULA WITHDRAWING DEVICE FOR INJECTION SYRINGES
DISPOSITIF DE RAPPEL AUTOMATIQUE DE LA CANULE DE SERINGUES D'INJECTION

(30) Priorität: 06.07.1995 CH 197395
(43) Veröffentlichungstag der Anmeldung: 24.11.1999
(73) Patentinhaber: Weidmann Plastics Technology AG, 8640 Rapperswil (CH); Vuille, Daniel, 4901 Langenthal (CH)
(72) Erfinder: VUILLE, Daniel, CH-4901 Langenthal (CH); BÜTTGEN, Heinz, CH-8645 Jona (CH); BURGER, Konrad, CH-8733 Eschenbach (CH); CALLENBACH, Tilo, CH-8645 Jona (CH); MAZENAUER, Karl, CH-8645 Jona (CH); RIESEN, Daniel, CH-8608 Bubikon (CH)
(74) Vertreter: Groner, Manfred
(86) Internationale Anmeldenummer: CH9600243
(87) Internationale Veröffentlichungsnummer: WO97002060

(56) Entgegenhaltungen:
- EP-A- 0 290 176
- EP-A- 0 566 882
- US-A- 5 114 404
- US-A- 5 180 369

## Beschreibung

Die Erfindung betrifft eine Kanüle mit einer Hohlnadel und einem Gehäuse sowie einer Einrichtung, mit der die Hohlnadel nach ihrer Benutzung in das Gehäuse zurückgezogen werden kann. Durch den vollständigen Rückzug der Hohlnadel nach deren Benutzung soll eine unbeabsichtigte Verletzung des Benutzers oder einer anderen Person vermieden werden.

Die US-A-5,114,404 offenbart eine Injektionsspritze mit einer Hohlnadel, die bei einer Benutzung zuerst ausgefahren und anschliessend wieder rückziehbar ist. Es hat sich gezeigt, dass bei einer solchen Injektionsspritze beim Rückzug der Hohlnadel Injektionsauslösung, die mit Blut des Patienten vermischt sein kann, durch die Hohlnadel ausgespritzt wird.

Die EP-A-0 290 176 offenbart eine Kanüle der genannten Gattung, bei welcher die Hohlnadel mittels einer Spiralfeder gespannt ist und durch Betätigung eines Schiebers in das Kanülengehäuse rückziehbar ist. Beim Rückzug der Hohlnadel wird im Kanülengehäuse Flüssigkeit verdrängt und durch die Hohlnadel ausgespritzt.

Durch die WO 92/16248 ist eine weitere Kanüle bekannt geworden. Diese ist eine Injektionskanüle und wird zum Gebrauch auf einen Spritzenzylinder aufgesetzt. Im Gehäuse ist die Hohlnadel mit einem Teil befestigt, das während der Injektion aufgelöst oder erweicht wird. Mit einer im Gehäuse gelagerten Druckfeder ist die Hohlnadel für den Rückzug gespannt. Ist der genannte Teil durch die Injektionsflüssigkeit aufgelöst oder erweicht, so ist die Fixierung der Hohlnadel im Gehäuse aufgehoben und diese wird durch die Druckfeder vollständig in das Gehäuse eingefahren. Bei dieser Kanüle besteht die Schwierigkeit, dass der Zeitpunkt der Auslösung des Rückzuges nicht genau definiert ist. Zudem ist es schwierig, eine Substanz zu finden, die einerseits durch die Injektionsflüssigkeit auflösbar oder erweichbar ist und andererseits völlig unbedenklich in die Injektionsflüssigkeit gelangen darf. In der Praxis hat sich diese Kanüle vermutlich aus diesen Gründen nicht durchgesetzt.

Eine weitere Kanüle dieser Gattung ist durch die WO 92/05818 bekannt geworden. Bei dieser ist die Hohlnadel an ihrem Ende an einem Spritzenzylinder angeformten Halteteil fixiert. Am Ende des Kolbenhubes wird dieses Halteteil mit einer am Kolben angebrachten Schneidkante vom Spritzenzylinder abgetrennt. Gleichzeitig wird mit der Schneidkante der Spritzenboden durchgetrennt. Der Rückzug erfolgt auch hier mittels einer im Gehäuse gelagerten Druckfeder. Versuche haben gezeigt, dass sehr hohe Kräfte erforderlich sind, um die beiden Teile aus Kunststoff und Gummi sauber zu durchtrennen. Bei dieser Kanüle widerspricht sich zudem die geforderte Dünnwandigkeit des Spritzenbodens und die geforderte Wandstärke gegen den Kaltfluss von Kunststoffteilen unter Belastung zum Abstützen der Federkraft. Die geforderte Wandstärke darf im Hinblick auf die vorgeschriebene lange Lagerfähigkeit der Kanüle ein bestimmtes Mass nicht unterschreiten.

Obwohl der dringende Bedarf nach einer solchen Kanüle nachgewiesen ist, scheint sich in der Praxis eine solche nicht bewährt zu haben.

Der Erfindung liegt die Aufgabe zugrunde, eine Kanüle der genannten Gattung zu schaffen, welche die oben genannten Nachteile vermeidet.

Die Aufgabe ist bei einer Kanüle gemäss Anspruch 1 gelöst. Bei der erfindungsgemässen Kanüle wird der Rückzug durch eine Umschaltvorrichtung ausgelöst. Mit einer solchen Umschaltvorrichtung kann der Auslösepunkt des Rückzugs genau bestimmt werden. Ein Durchtrennen von Kunststoffteilen oder ein Auflösen von Substanzen mittels Injektionsflüssigkeit ist bei der erfindungsgemässen Kanüle nicht erforderlich. Die Auslösekraft kann sehr klein gehalten werden und die Konstruktion erlaubt eine materialgerechte Bauweise.

Die erfindungsgemässe Kanüle ist insbesondere als Injektionskanüle für eine Spritze vorgesehen. Sie kann einen Luer-Ansatz aufweisen und beim Aufstecken auf die Spritze unlösbar mit dieser verbunden werden. Die Kanüle kann aber auch ursprünglich fest mit der Spritze verbunden sein.

Wesentlich ist, dass bei der erfindungsgemässen Kanüle die üblichen Normvorschriften erfüllt werden können. Daraus ergibt sich eine Komptabilität mit Standardkanülen und Standardspritzen für den Notfall, d.h. wenn umständehalber keine erfindungsgemässen Spritzen oder Kanülen vorhanden sind. Zudem kann die Umschaltvorrichtung in einem vergleichsweise kleinen Gehäuse untergebracht werden, sodass die Kanüle äusserlich kaum von einer üblichen Kanüle unterscheidbar ist. Wie im Spital üblich, kann eine Aufzugskanüle durch die erfindungsgemässe Kanüle ausgewechselt werden. Wie erwähnt, ist jedoch auch eine Ausführung denkbar, bei der die erfindungsgemässe Kanüle unlösbar mit dem Spritzenzylinder verbunden ist.

Eine konstruktiv besonders einfache Realisierung der Umschaltvorrichtung ist nach einer Weiterbildung der Erfindung dann gegeben, wenn diese ein im Gehäuse drehbar gelagertes Umschaltteil aufweist, an dem ein mit der Hohlnadel fest verbundener Halter abgestützt ist. Bei der Betätigung der Umschaltvorrichtung wird das Umschaltteil in eine Drehposition gedreht, in welcher der Halter mit der Hohlnadel in das Gehäuse rückziehbar ist. Die Rückziehbewegung kann beispielsweise mit einer Druckfeder erfolgen. Denkbar sind auch andere Energiespeicher.

Die Umschaltvorrichtung weist ein Ausgleichsteil auf, mit der zur Aufnahme der beim Rückzug der Hohlnadel verdrängten Flüssigkeit während des Umschaltvorgangs ein Unterdruck in einer Injektionsflüssigkeit erzeugbar ist. Damit kann vermieden werden, dass beim Rückzug der Hohlnadel Injektionslösung, die mit Blut des Patienten vermischt sein kann, durch die Hohlnadel ausgespritzt wird. Mit dem Ausgleichsteil wird somit ein Volumenausgleich geschaffen, der es ermöglicht, dass beim Rückzug der Hohlnadel keine verdrängte Flüssigkeit nach aussen abgegeben wird.

Die Erfindung betrifft auch eine Injektionsspritze nach Anspruch 20. Bei einer solchen Injektionsspritze kann die Umschaltvorrichtung durch einen Kolbenhub ausgelöst werden. Die Mittel können beispielsweise einen Stift aufweisen, der nach der Injektion in den Ansatz des Spritzzylinders eingreift. Die Umschaltvorrichtung wird vorzugsweise etwa dann ausgelöst, wenn die Kolbendichtung bis auf den Spritzboden durchgedrückt ist. Diese Injektionsspritze kann mit einer Hand, wie sonst üblich, bedient werden, was in der Praxis ein wesentlicher Vorteil ist. Der Stift ist vorzugsweise eine Verlängerung der Kolbenstange.

Weiter vorteilhafte Merkmale ergeben sich aus den abhängigen Patentansprüchen, der nachfolgenden Beschreibung sowie der Zeichnung. Ein Ausführungsbeispiel der Erfindung wird nachfolgend anhand der Zeichnung näher erläutert. Es zeigen:
- Figur 1: ein Längsschnitt durch eine erfindungsgemässe Kanüle sowie einen Ansatz einer Injektionsspritze,
- Figur 2: ein Schnitt gemäss Figur 1, jedoch nach dem Rückzug der Hohlnadel,
- Figur 3: ein Querschnitt durch die Kanüle entlang der Linie III-III der Figur 1,
- Figur 4: ein Querschnitt durch die Kanüle entlang der Linie IV-IV der Figur 1,
- Figur 5: ein Querschnitt durch die Kanüle entlang der Linie V-V der Figur 1,
- Figur 6: ein Querschnitt durch die Kanüle entlang der Linie VI-VI der Figur 1,
- Figur 7: ein Querschnitt durch die Kanüle entlang der Linie VII-VII der Figur 2, und
- Figur 8: ein Längsschnitt durch eine erfindungsgemässe Injektionsspritze und einen Ansatz einer erfindungsgemässen Kanüle.

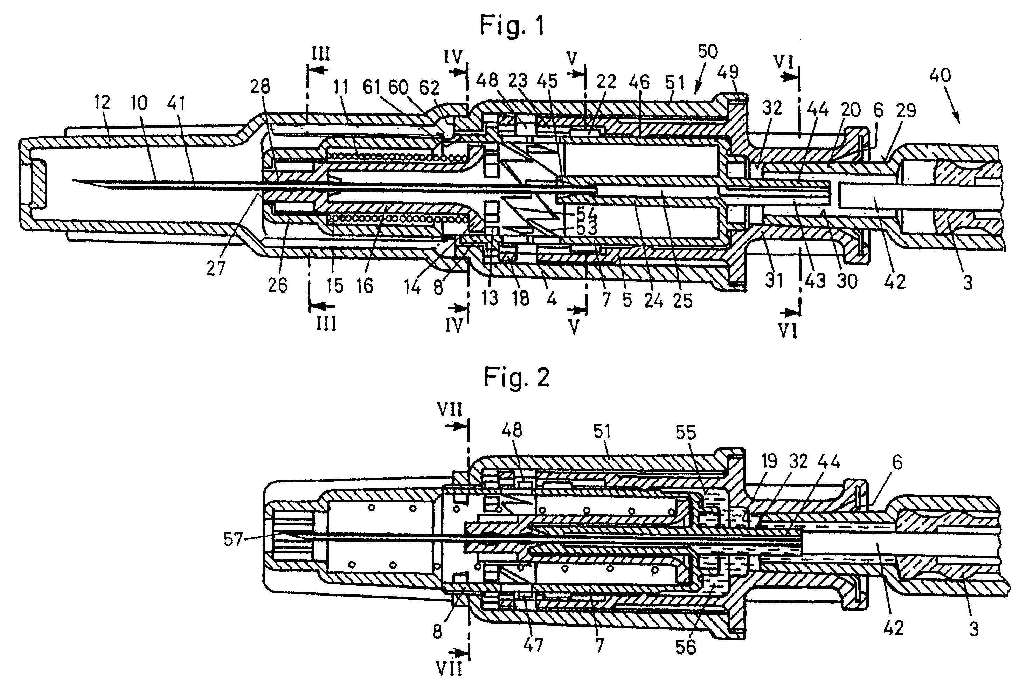

Die Figur 1 zeigt einen konischen Ansatz 20 am Gehäuse 63 einer Injektionsspritze 40, auf den eine gebrauchsfertige erfindungsgemässe Kanüle 50 aufgesetzt ist. Der Ansatz 20 ist vorzugsweise ein Luer-Ansatz mit einem Aussenkegel 29. Korrespondierend zu diesem weist ein hinteres Gehäuseteil 5 einen Ansatz 31 mit einem korrespondierenden Innenkegel 32 auf. Am hinteren Ende des Ansatzes 31 ist an diesem ein nach innen ragender Sicherungsring 6 angebracht, der beim Aufsetzen der Kanüle 50 auf die Injektionsspritze 40 die Kanüle 50 unlösbar mit dem Ansatz 20 verbindet. Denkbar ist jedoch auch eine Ausführung, bei welcher die Kanüle 50 lösbar mit der Injektionsspritze 40 verbunden ist.

Das hintere Gehäuseteil 5 greift in ein vorderes Gehäuseteil 4 ein und bildet mit diesem das Gehäuse 51. Beide Gehäuseteile sind beispielsweise an einer umlaufenden Schweissstelle 49 unlösbar miteinander verbunden. An einem vorderen Ende 26 des vorderen Gehäuseteils 4 ist abnehmbar eine Schutzkappe 12 befestigt. Diese wird vor der Benützung der Kanüle 50 abgenommen und deckt das vorragende Ende einer Hohlnadel 10 ab. Die Schutzkappe 12 besitzt innenseitig mehrere Nocken 60, die jeweils in einen Schlitz 61 einer Schalthülse 8 eingreifen, und diese gegen ein Verdrehen sichern. Für den Eingriff der Nocken 60 ist das vordere Gehäuseteil 4 mit Längsschlitzen 62 versehen. Die Schutzkappe 12 bildet damit eine Transportsicherung.

Die Hohlnadel 10 ist mittig in einem Durchgangsloch 28 eines Nadelhalters 16 unverschiebbar gehalten und gegen Verdrehen gesichert. Der Nadelhalter 16 greift mit seinem vorderen Ende in eine Oeffnung 27 des vorderen Gehäuseteils 4 ein und ist in dieser Oeffnung 27 gegen Verdrehen gesichert. Am hinteren Ende sind am Nadelhalter 16 mehrere radial nach aussen ragende Nocken 14 angeformt, die auf radial nach innen ragenden Nocken 13 einer Schalthülse 8 aufliegen. Eine gespannte Druckfeder 11, die an einem vorderen Ende an einer Schulter 15 des Gehäuseteils 4 und am verbreiterten hinteren Ende des Nadelhalters abgestützt ist, spannt den Nadelhalter 16 axial nach hinten gegen die Schalthülse 8. Diese ist im vorderen Gehäuseteil 4 begrenzt drehbar gelagert und an einem vorderen Rand 18 des hinteren Gehäuseteils 5 abgestützt. Im Bereich des Randes 18 besitzt das hintere Gehäuseteil 5 zwei gegenüberliegende Aussparungen 48, in die jeweils ein Nocken 47 (Figur 2) der Schalthülse 8 eingreift. Mit den Nocken 47 ist die Schalthülse 8 begrenzt verdrehbar auf dem hinteren Gehäuseteil 5 positioniert. Die Schalthülse 8 ist somit axial fixiert, jedoch radial begrenzt verdrehbar.

Im Gehäuseteil 5 ist ein Ausgleichsteil 7 gelagert, das mit seitlich vorspringenden Nocken 22 in die Führungsnuten 23 des Gehäuseteils 5 eingreifen, wodurch ein Verdrehen gegenüber dem Gehäuse 51 verhindert ist. Die Führungsnuten 23 erlauben jedoch eine Verschiebung des Ausgleichsteils 7 nach vorne gegen die Schalthülse 8 hin. Eine umlaufende Dichtrippe 46 an der Innenseite des hinteren Gehäuseteils 5 dichtet das Ausgleichsteil 7 gegen das Gehäuse 51 ab. Die Dichtwirkung wird beim Verschieben des Ausgleichsteils 7 aufrechterhalten, kann aber auch gegen Ende des Verschiebungsweges geöffnet sein. In das Innere des becherförmigen Ausgleichsteils 7 ragt ein rohrförmiger Führungsteil 24 mit einem durchgehenden Kanal 25, in welchen der hintere Teil der Hohlnadel 10 eingreift. Eine sich verengende vordere Oeffnung 45 des Kanals 25 dichtet die Aussenseite der Hohlnadel 10 gegen das Ausgleichsteil 7 ab. Die Oeffnung 45 erlaubt dennoch eine Verschiebung der Hohlnadel 10 im Kanal 25. Der Kanal 25 mündet nach rückwärts in einen hinteren, rohrförmigen Ansatz 44, der auf seiner gesamten Länge radial durchgehende Schlitze 43 aufweist. Der Kanal 25 ist somit im Bereich des Ansatzes 44 seitlich offen. Wie die Figur 1 deutlich zeigt, ragt der Ansatz 44 in den Innenkegel 32 und in das Durchgangsloch 30 des Ansatzes 20.

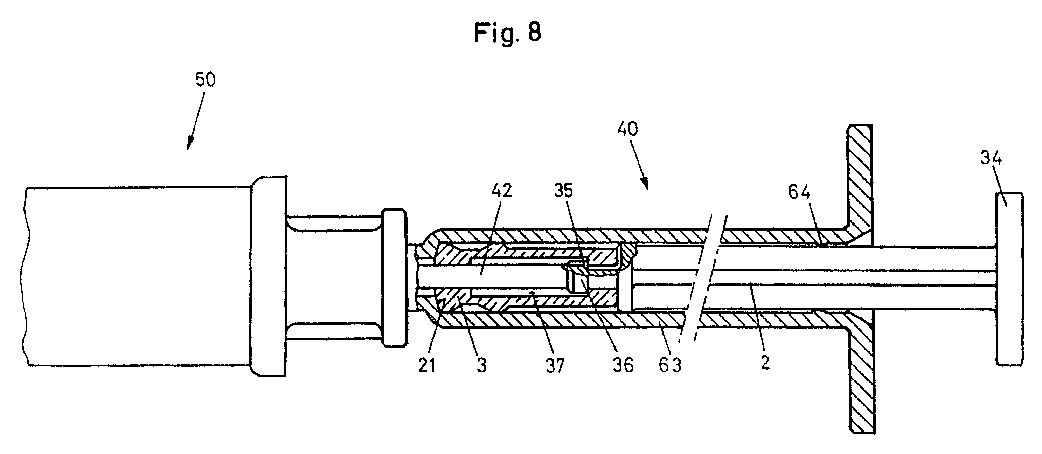

Das Ausgleichsteil 7 kann aus der in Figur 1 gezeigten Position mit einem Stift 42 der Injektionsspritze 40 in die in Figur 2 gezeigte Position verschoben werden. Der Stift 42 ist gemäss Figur 8 am vorderen Ende einer Kolbenstange 2 der Spritze 40 angeordnet und vorzugsweise eine Fortsetzung der Kolbenstange 2. Die Kolbenstange 2 ist mit einer üblichen Druckplatte 34 versehen. Die Figur 8 zeigt die Kolbenstange 2 im eingedrückten Zustand nach einer Injektion.

Nachfolgend wird die Funktionsweise der Kanüle näher erläutert.

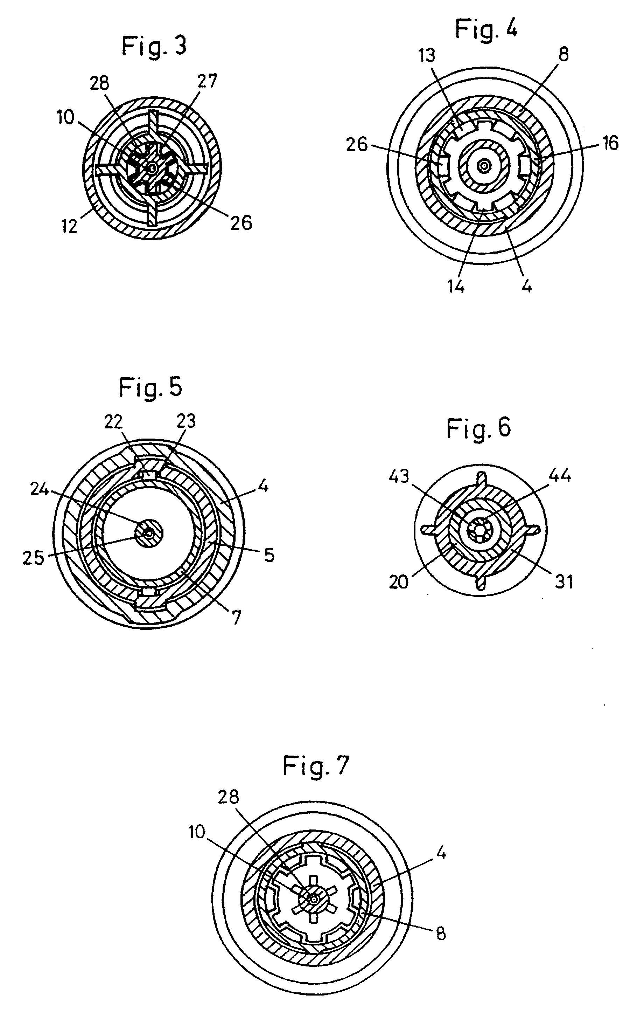

Die Injektionsspritze 40 wird mit einer hier nicht gezeigten Aufziehkanüle, die auf dem Ansatz 20 aufgesetzt wird, mit Injektionsflüssigkeit 19 gefüllt. Danach wird die Aufziehkanüle durch die Injektionskanüle 50 ausgewechselt und die Schutzkappe 12 wird abgezogen. Anschliessend kann mit einem kurzen Hub des zurückgezogenen Kolbens 3 zur Verhinderung einer Mikroembolie Luft aus der Kanüle 50 ausgestossen werden. Die Hohlnadel 10 kann nun in ein Gewebe des Patienten eingeführt werden. Die Injektionsflüssigkeit 19 wird wie üblich durch Verschieben des Kolbens 3 im Spritzenzylinder 1 eingespritzt. Die Injektionslösung 19 verlässt hierbei den Spritzenzylinder 1 und die Kanüle 50 durch die Durchgangslöcher bzw. Kanäle 30, 25 und 41 der Hohlnadel 10. Kurz vor Beendigung der Injektion stösst der Stift 42 an den Ansatz 44 und verschiebt dadurch das Ausgleichsteil 7 in Längsrichtung der Kanüle 50 gegen die Schalthülse 8. Das gegen Verdrehen gesicherte Ausgleichsteil 7 verdreht hierbei die Schalthülse 8 durch einen Eingriff einer stirnseitig angebrachten Verzahnung 53 mit einer korrespondierenden Verzahnung 54 der Schalthülse 8. Gleichzeitig wird durch die Verschiebung des Ausgleichsteils 7 zwischen diesem und der Innenseite 55 des hinteren Gehäuseteils 5 ein ringförmiger Zwischenraum 56 geschaffen, durch den in der Injektionsflüssigkeit 19 ein Unterdruck erzeugt wird. Durch das Verdrehen der Schalthülse 8 werden die Nocken 13 in Figur 4 ebenfalls verdreht und die Nocken 14 des Nadelhalters 16 kommen über Lücken 17 zwischen den Nocken 13 zu liegen. Die Abstützung des Nadelhalters 16 an der Schalthülse 8 ist damit aufgehoben und der Nadelhalter 16 wird durch die gespannte Druckfeder 11 zusammen mit der Hohlnadel 10 in die in Figur 2 gezeigte Position zurückgezogen. Wie ersichtlich, ist nun die Hohlnadel 10 mit der vorderen Spitze 57 vollständig im vorderen Gehäuseteil 4 untergebracht. Der während der Verschiebung des Ausgleichsteils 7 geschaffene Raum 56 nimmt die durch den Rückzug der Hohlnadel 10 verdrängte Flüssigkeit auf, wodurch ein Verspritzen von Flüssigkeit durch das Durchgangsloch 41 vermieden wird.

Wie die Figur 8 zeigt, ist der Kolben 3 hülsenförmig ausgebildet und an einem hinteren Ende an einem Kopf 36 der Kolbenstange 2 befestigt. Der aus gummielastischem Material bestehende Kolben 3 kann aus der in Figur 8 gezeigten Lage zur Ausführung eines weiteren Hubes wie ein Faltenbalg axial zusammengedrückt werden, wobei in einem Durchgang 37 vorhandene Luft durch eine Luftnut 35 nach hinten ausgestossen wird. Mit diesem weiteren Hub wird das Ausgleichsteil 7 verschoben.

Ausserdem kann dadurch ein Längenausgleich, welcher sich durch unterschiedliche Aufstecklängen ergibt, kompensiert werden.

Die in das Gehäuse 51 zurückgezogene Hohlnadel 10 kann weder absichtlich noch unabsichtlich wieder in die in Figur 1 gezeigte Position gebracht werden. Einerseits ist die Hohlnadel 10 von aussen kaum zugänglich und andererseits müsste diese gleichzeitig gegen die rückwirkende Kraft der Druckfeder 11 nach vorne bewegt und das Umschaltteil müsste in gespannter Position der Feder gedreht werden. Durch eine feste Verbindung der Kanüle 50 am Ansatz 20 der Spritze 40, beispielsweise mittels eines Sicherungsrings 6, kann ein solches Zurückschieben der Hohlnadel 10 weiter erschwert werden. Eine unlösbare Verbindung sichert auch die Spritze vor einem Wiedergebrauch. Eine innenseitig am Gehäuse 63 angeformte Rippe 64 bildet eine Sperre gegen ein Ausziehen der Kolbenstange 2.

Die Kanüle 50 besteht aus vergleichsweise wenigen Teilen. Kunststoffteile lassen sich aus medizinisch zulässigen Werkstoffen, insbesondere Polypropylen oder Polyethylen in Spritzgussverfahren herstellen. Die Nadel kann aus genormtem Medizinalstahl gefertigt sein. Ein vergleichsweise einfacher Zusammenbau der Einzelteile ist möglich und auch automatisierbar. Bei der erfindungsgemässen Kanüle 50 sind nicht nur die üblichen Normen erfüllbar, sondern ist auch eine kostengünstige Serienherstellung möglich. Schliesslich ist die Bedienbarkeit mit einer üblichen Spritze ohne Rückzug der Hohlnadel vergleichbar. Insbesondere ist eine Einhandbedienung möglich und es sind für den Rückzug der Hohlnadel keine wesentlichen Kräfte erforderlich. Die Auslösung des Rückzugs ist genau bestimmt und kann mechanisch festgelegt werden. Wesentlich ist zudem, dass der Rückzug der Hohlnadel 10 nach einer Injektion mit hoher Sicherheit ausgelöst wird.

## Patentansprüche

1. Kanüle mit einer Hohlnadel (10) und einem Gehäuse (51) sowie einer Einrichtung, mit der die Hohlnadel (10) nach ihrer Benutzung in das Gehäuse (51) rückziehbar ist, wobei die Einrichtung eine Umschaltvorrichtung (7, 8) aufweist, die nach der Benutzung der Hohlnadel (10) für deren Rückzug schaltbar ist, **dadurch gekennzeichnet, dass** die Umschaltvorrichtung ein Ausgleichsteil (7) aufweist, mit dem zur Aufnahme der bei Rückzug der Hohlnadel (10) verdrängten Flüssigkeit während des Umschaltvorgangs ein Unterdruck erzeugt wird.

2. Kanüle nach Anspruch 1, **gekennzeichnet durch** einen im Gehäuse (51) drehbar gelagerten Umschaltteil (8), an dem ein mit der Hohlnadel (10) fest verbundener Halter (16) abgestützt ist.

3. Kanüle nach Anspruch 2, **dadurch gekennzeichnet, dass** der Halter (16) die Hohlnadel (10) im Abstand zum hinteren Ende der Hohlnadel (10) festhält.

4. Kanüle nach Anspruch 2, **dadurch gekennzeichnet, dass** der Halter (16) mit einem Energiespeicher, insbesondere einer Druckfeder (11) gegen den Umschaltteil (8) gespannt ist.

5. Kanüle nach einer der Ansprüche 2 bis 4, **dadurch gekennzeichnet, dass** der Halter (16) an seinem hinteren Ende mehrere nach aussen vorstehende Nocken (14) aufweist, die an vorstehenden Nocken (13) des Umschaltteils (8) abgestützt sind.

6. Kanüle nach einem der Ansprüche 2 bis 5, **dadurch gekennzeichnet, dass** das Umschaltteil (8) hülsenförmig ausgebildet ist.

7. Kanüle nach Anspruch 1, **dadurch gekennzeichnet, dass** das Ausgleichsteil (7) ein Führungsteil (24) zur Aufnahme des hinteren Endes der Hohlnadel (10) besitzt.

8. Kanüle nach Anspruch 1 oder 2, **dadurch gekennzeichnet, dass** mit dem Ausgleichsteil (7) das Umschaltteil (8) umschaltbar ist.

9. Kanüle nach Anspruch 2, **dadurch gekennzeichnet, dass** das Ausgleichsteil (7) im Gehäuse (51) verschiebbar gelagert ist und beim Verschieben des Ausgleichsteils (7) dieser zum Drehen des Umschaltteils (8) mit diesem in Eingriff bringbar ist.

10. Kanüle nach einem der Ansprüche 1 bis 9, **dadurch gekennzeichnet, dass** das Gehäuse (51) ein vorderes und ein hinteres Gehäuseteil (4, 5) aufweist, die zusammengebaut und unlösbar miteinander verbunden sind.

11. Kanüle nach Anspruch 10, **dadurch gekennzeichnet, dass** das hintere Gehäuseteil (5) in das vordere Gehäuseteil (4) eingreift und dass am hinteren Gehäuseteil (5) das Umschaltteil (8) drehbar abgestützt ist.

12. Kanüle nach Anspruch 10 oder 11, **dadurch gekennzeichnet, dass** am hinteren Gehäuseteil (5) das Ausgleichsteil (7) axial nach vorne schiebbar und drehsicher gelagert ist.

13. Kanüle nach einem der Ansprüche 10 bis 12, **dadurch gekennzeichnet, dass** zum Umschalten des Ausgleichsteils (7) im Gehäuse (51) axial nach vorne verschiebbar ist.

14. Kanüle nach einem der Ansprüche 10 bis 13, **dadurch gekennzeichnet, dass** das Gehäuse (51) Mittel (5) zu seiner unlösbaren Befestigung an einer Vorrichtung (40) aufweist.

15. Kanüle nach einem der Ansprüche 10 bis 14, **dadurch gekennzeichnet, dass** das Gehäuse (51) an einem hinteren Ende einen Innenkonus (32) aufweist, in dem ein am Ausgleichsteil (7) angebrachter Ansatz (44) ragt, an dem das Ausgleichsteil (7) betätigbar ist.

16. Kanüle nach einem der Ansprüche 1 bis 15, **gekennzeichnet durch** eine Schutzkappe (12) die Mittel (60) aufweist, die zur Transportsicherung mit der Umschaltvorrichtung (7, 8) ein Eingriff ist und einen unbeabsichtigten Rückzug der Hohlnadel verhindert.

17. Kanüle nach Anspruch 16, **dadurch gekennzeichnet, dass** die Schutzkappe (12) vorstehende Nocken (60) aufweist, die durch Ausnehmungen (62) des Gehäuses (51) der Kanüle hindurchgreifen.

18. Kanüle nach einem der Ansprüche 1 bis 17, **dadurch gekennzeichnet, dass** ein Nadelhalter (16) durch einen Eingriff am Gehäuse (51) der Kanüle gegen Verdrehen gesichert ist.

19. Kanüle nach einem der Ansprüche 1 bis 18, **dadurch gekennzeichnet, dass** die Kanüle Mittel (22, 23, 47, 48) zur Positionierung von Teilen (4, 5, 7) insbesondere während der Montage aufweist.

20. Injektionsspritze mit einer Kanüle gemäss Anspruch 1 mit einem Spritzenzylinder (40) und einer in diesem gelagerten Kolbenstange (1) mit einem Kolben (3) am vorderen Ende und einem Aussenkonus (29), an dem eine Kanüle (50) gemäss Anspruch 1 angeschlossen oder anschliessbar ist, **dadurch gekennzeichnet, dass** am Kolben (3) Mittel (42) zur Betätigung der Umschaltvorrichtung der Kanüle (50) angeordnet sind.

21. Spritze nach Anspruch 20, **dadurch gekennzeichnet, dass** die Mittel (42) zur Betätigung der Umschaltvorrichtung einen Stift aufweisen, der an der Kolbenstange (2) angebracht ist und der nach eine Injektion in einen Ansatz (20) des Spritzenzylinders (40) eingreift.

22. Spritze nach Anspruch 20 oder 21, **dadurch gekennzeichnet, dass** der Kolben (3) eine axial zusammendrückbare Hülse aufweist, die an einem vorderen Ende am Spritzenboden (21) anlegbar ist.

23. Spritze nach Anspruch 23, **dadurch gekennzeichnet, dass** die Kolbenstange (2) das hintere Ende des Stiftes (42) aufnimmt.

24. Spritze nach einem der Ansprüche 20 bis 23, **dadurch gekennzeichnet, dass** der Kolben (3) so ausgebildet ist, dass er einen Längenausgleich bildet, welcher eine Auslösung auch bei unterschiedlichen Aufstecklängen ermöglicht, welche sich beispielsweise durch Massabweichungen ergeben.

## Claims

1. Cannula consisting of a hollow needle (10), a housing (51) and a device y means of which the hollow needle (10), after use, can be retracted into the housing (51), wherein the device incorporating a selector switch device (7, 8) which can be switched for retraction of the hollow needle (10) after its use, **characterised by** the selector switch device incorporating an equalization part (7) and by which during the selector switch process a negative pressure is produced to accept the fluid compressed during the retraction of the hollow needle (10).

2. Cannula as per Claim 1 **characterized by** a rotatable selector switch part (8) located in the housing (51) on which a mount (16) permanently connected to the hollow needle (10) is supported.

3. Cannula as per Claim 2 thereby **characterized by** the mount (16) permanently holding the hollow needle (10) at a distance from the rear end of the hollow needle (10).

4. Cannula as per Claims 2 and 3 thereby **characterized by** the mount (16) being stressed by an energy source, especially a pressure spring (11), against the selector switch part (8).

5. Cannula as per Claims 2 to 4 thereby **characterized by** the mount incorporation several outward projecting cams (14) on its rear end which are supported on protruding cams (13) of the selector switch part (8).

6. Hollow needle as per Claims 2 to 5 thereby **characterized by** the selector switch part (8) being shaped like a case.

7. Hollow needle as per Claim 1 thereby **characterized by** the equalization part (7) possessing a guide part (24) to accept the rear part of a hollow needle (10).

8. Hollow needle as per Claims 7 or 8 thereby **characterized by** the selector switch part (8) being switched by the equalization part (7).

9. Cannula as per Claim 2 thereby **characterized by** the displaceable equalization part (7) being located in the housing (51) and during displacement of the equalization part (7) engaging the selector switch part (8) in order to turn it.

10. Cannula as per Claims 1 to 9 thereby **characterized by** the housing (51) manifesting a front and a rear housing part (4, 5) which are assembled and permanently joined together.

11. Cannula as per Claim 10 thereby **characterized by** the rear housing part (5) engaging the front housing part (4) and the rotatable selector switch part (8) being supported on the rear housing part (8).

12. Cannula as per Claim 10 or 11 thereby **characterized by** the equalization part (7) on the rear housing part (5) being positioned so that it axially displaces to the front and rotates.

13. Cannula as per Claims 10 to 12 thereby **characterized by** the equalization part (7) displacing axially to the front in the housing (51) for switching.

14. Cannula as per Claims 10 to 13 thereby **characterized by** the housing (51) manifesting means (6) of its inseparable connection to a device (40).

15. Cannula as per Claims 10 to 14 thereby **characterized by** the housing (51) incorporating an inward cone (32) on a rear end into which extends a projection (44) attached to the equalization part (7) in order to activate the equalization part (7).

16. Cannula as per Claims 1 to 15 thereby **characterized by** a protective cap (12) incorporating the means (6) which for transport safety engages the selector switch device (7, 8) and prevents an unintentional retraction of the hollow needle.

17. Cannula as per Claim 16 thereby **characterized by** the protective cap (12) incorporating protruding cams (60) which engage into recesses (62) on the housing (51) of the hollow needle.

18. Cannula as per one of the Claims 1 to 17 thereby **characterized by** the needle mount (16) preventing any turning by engaging the housing (51) of the hollow needle.

19. Cannula as per Claims 1 to 18 thereby **characterized by** the hollow needle manifesting the means (22, 23, 47, 48) to position parts (4, 5, 7) especially during assembly.

20. Injection syringe with a cannula according to Claim 1 with a needle cylinder (40) and a piston rod (1) stored therein with a piston (3) on the front end and an exterior cone (29) on which a hollow needle (50) is, or can be, attached as per Claim 1 thereby **characterized by** means to activate the selector switch device of the hollow needle (50) being attached.

21. Injection syringe as per Claim 21 thereby **characterized by** the means (42) incorporating a pin to activate the selector switch device which is attached to the piston rod and which after injection engages a projection (20) of the hypodermic cylinder (40).

22. Injection syringe as per Claims 21 or 22 thereby **characterized by** the piston (3) manifesting an axially compressible casing whose front end can be place on the hypodermic needle cylinder base (21).

23. Injection syringe as per Claim 22 thereby **characterized by** the piston rod (2) accepting the rear end of the pin (42).

24. Injection syringe as per one of the Claims 20 to 23 thereby **characterized by** the piston (3) being so formed that a longitudinal equalization occurs which makes possible initiation for varying emplacement lengths which can result, for example, from deviations of the dimensions.

## Revendications

1. Canule comportant une aiguille creuse (10) et un boîtier (51) ainsi qu'un dispositif, au moyen duquel l'aiguille creuse (10) peut être rétractée dans le boîtier (51) après son utilisation, le dispositif comportant un dispositif de commutation (7, 8), qui après l'utilisation de l'aiguille creuse (10), peut être commuté pour le retrait de l'aiguille, **caractérisée en ce que** le dispositif de commutation comporte une partie de compensation (7), avec laquelle se produit une dépression pour la réception du liquide refoulé lors du retrait de l'aiguille creuse (10) pendant l'opération de commutation.

2. Canule selon la revendication 1, **caractérisée par** une partie de commutation (8), qui est montée de manière à pivoter dans le boîtier (51) et sur laquelle prend appui un dispositif de retenue (16) relié de façon fixe à l'aiguille creuse (10).

3. Canule selon la revendication 2, **caractérisée en ce que** le dispositif de retenue (16) maintient fermement l'aiguille creuse (10) à distance de l'extrémité arrière de l'aiguille creuse (10).

4. Canule selon la revendication 2, **caractérisée en ce que** le dispositif de retenue (16) est serré contre la partie de commutation (8) au moyen d'un accumulateur d'énergie, notamment d'un ressort de compression (11).

5. Canule selon l'une des revendications 2 à 4, **caractérisée en ce que** le dispositif de retenue (16) comporte, sur son extrémité arrière, plusieurs ergots (14) qui font saillie vers l'extérieur et qui prennent appui sur des ergots avant (13) de la partie de commutation (8).

6. Canule selon les revendications 2 à 5, **caractérisée en ce que** la partie de commutation (8) est agencée en forme de douille.

7. Canule selon la revendication 1, **caractérisée en ce que** la partie de compensation (7) possède une partie de guidage (24) servant à recevoir l'extrémité arrière de l'aiguille creuse (10).

8. Canule selon la revendication 1 ou 2, **caractérisée en ce que** la partie de commutation (8) peut être commutée avec la partie de compensation (7).

9. Canule selon la revendication 2, **caractérisée en ce que** la partie de compensation (7) est montée de mobile dans le boîtier (51) et, lors du déplacement de la partie de compensation (7), cette dernière vient en prise avec la partie de commutation (8) pour la faire tourner.

10. Canule selon l'une des revendications 1 à 9, **caractérisée en ce que** le boîtier (51) possède une partie avant et une partie arrière (4, 5), qui sont assemblées et reliées entre elles de façon inamovible.

11. Canule selon la revendication 10, **caractérisée en ce que** la partie arrière (5) du boîtier s'engage dans la partie avant (4) du boîtier et que la partie de commutation (8) prend appui à rotation sur la partie arrière (5) du boîtier.

12. Canule selon la revendication 10 ou 11, **caractérisée en ce que** la partie de compensation (7) est montée mobile axialement vers l'avant tout en étant bloquée en rotation sur la partie arrière (5) du boîtier.

13. Canule selon l'une des revendications 10 à 12, **caractérisée en ce que** pour la commutation, la partie de compensation (7), est déplaçable axialement vers l'avant dans le boîtier (51).

14. Canule selon l'une des revendications 10 à 13, **caractérisée en ce que** le boîtier (51) comporte des moyens (5) pour sa fixation inamovible à un dispositif (43).

15. Canule selon l'une des revendications 10 à 14, **caractérisée en ce que** le boîtier (51) comporte, sur une extrémité arrière, un cône intérieur (32), dans lequel s'engage un embout (44) monté sur la partie de compensation (7) et auquel la partie de compensation (7) peut être fixée.

16. Canule selon l'une des revendications 1 à 15, **caractérisée par** un capuchon de protection (12) qui comporte des moyens (60) en prise avec le dispositif de commutation (7, 8) pour la sécurité du transport et empêchant un retrait intempestif de l'aiguille creuse.

17. Canule selon la revendication 16, **caractérisé en ce que** le capuchon de protection (12) comporte des ergots saillants (60), qui traversent des évidements (62) du boîtier (51) de la canule.

18. Canule selon l'une des revendications 1 à 17, **caractérisée en ce qu'**un dispositif (16) de retenue d'aiguille est bloqué en rotation par une prise avec le boîtier (51) de la canule.

19. Canule selon l'une des revendications 1 à 18, **caractérisée en ce que** la canule comporte des moyens (22, 23, 47, 48) pour positionner des parties (4, 5, 7) notamment pendant le montage.

20. Seringue d'injection comportant une canule selon la revendication 1, comportant un cylindre de seringue (40) et une tige de piston (1) montée dans ce cylindre et comportant un piston (3) situé à l'extrémité avant et un cône extérieur (29), auquel la canule (50) selon la revendication 1 est ou peut être raccordée, **caractérisée en ce que** les moyens (42) pour actionner le dispositif de commutation de la canule (5) sont disposés sur le piston (3).

21. Seringue selon la revendication 1, **caractérisé en ce que** les moyens (42) pour actionner le dispositif de commutation possèdent une tige qui est montée sur la tige de piston (2) et qui, après une injection, s'engagent dans un embout (20) du cylindre (40) de la seringue.

22. Seringue selon la revendication 20 ou 21, **caractérisée en ce que** le piston (3) comporte une douille pouvant être comprimée axialement et qui peut être appliquée sur une extrémité avant du fond (21) de la seringue.

23. Seringue selon la revendication 22, **caractérisée en ce que** la tige de piston (2) loge l'extrémité arrière de la tige (42).

24. Seringue selon l'une des revendications 20 à 23, **caractérisée en ce que** le piston (3) est agencé de manière à compenser les écarts de longueur résultant notamment d'écarts de cotes.
